# EUROPÄISCHE PATENTANMELDUNG

(11) **EP 0 591 613 A1**
(43) Veröffentlichungstag der Anmeldung: **13.04.1994**
(21) Anmeldenummer: 93107768.9
(22) Anmeldetag: 13.05.1993
(51) Int. Cl.: A61F 7/12, A61B 17/22, A61B 19/08, A61C 19/00, A61C 1/16

(54) **Schutzvorrichtung für ein medizinisches Bestrahlungsgerät**

(30) Priorität: 08.10.1992 DE 4233870
(71) Anmelder: Heraeus Kulzer GmbH, 63450 Hanau (DE)
(72) Erfinder: Holtz, Herwig, Dr., W-6382 Friedrichsdorf 3 (DE); Fritze, Joachim, Dr., W-6382 Friedrichsdorf 3 (DE)

(57) **Zusammenfassung**

Die Erfindung betrifft eine Schutzvorrichtung für ein medizinisches Bestrahlungsgerät, die ein Hüllteil mit einer Aufnahmeöffnung und mit Mitteln zur Halterung des Hüllteils an einem Bestrahlungsgerät aufweist. Um eine Schutzvorrichtung bereitzustellen, die eine Übertragung von Keimen bzw. Verunreinigungen vom Patienten auf das Gerät sowie umgekehrt von dem Gerät auf den Patienten zuverlässig verhindert, ohne die Funktionsweise des Bestrahlungsgerätes wesentlich zu beeinträchtigen, ist wenigstens an dem geschlossenen Ende des Hüllteils ein Fenster vorgesehen, das für Strahlung im Wellenlängenbereich von 370-520 nm wenigstens teilweise durchlässig ist.

## Beschreibung

Die Erfindung betrifft eine Schutzvorrichtung für ein medizinisches Bestrahlungsgerät, die ein Hüllteil mit einer Aufnahmeöffnung und mit Mitteln zur Halterung des Hüllteils an einem Bestrahlungsgerät aufweist.

Vorrichtungen zum Schutz von medizinischen Geräten im allgemeinen und von medizinischen Bestrahlungsgeräten im besonderen sind allgemein bekannt. Diese Vorrichtungen dienen im wesentlichen dem Schutz der Geräte vor Verunreinigungen, insbesondere mit Krankheitserregern. Eine solche Schutzvorrichtung für ein medizinisches Bestrahlungsgerät ist aus der DE-A1 39 01 199 bekannt. Hier wird eine schwenkbare zahnärztliche Behandlungsleuchte beschrieben, deren Leuchtengriff von einer Schutzhülle überzogen ist. Diese Schutzhülle verhindert, daß bei Berührung des Leuchtengriffes durch den behandelnden Zahnarzt Keime aus dem Mund des Patienten auf die Behandlungsleuchte bzw. von dort auf den nachfolgenden Patienten übertragen werden. Die Schutzhülle kann nach jeder Behandlung durch eine neue ausgetauscht werden und ersetzt dadurch die aufwendige Sterilisation des Handgriffes.

Eine ähnliche Schutzvorrichtung ist auch aus der US 4,605,124 bekannt. Hier wird eine Operationsleuchte beschrieben mit einem zentral an der Lichtsaustrittsfläche angeordneten Handgriff. Dieser Handgriff wird zum Schutz vor Kontamination von einem Überzug umgeben, der zum Anschluß an das Lampengehäuse hin von einer tellerartigen Erweiterung abgeschlossen wird. Dieser Schutzüberzug über den Handgriff verhindert ebenfalls eine Übertragung von Keimen oder anderen Verunreinigungen von der Hand des behandelnden Arztes auf die Leuchte, so daß ein umständliches Desinfizieren der Leuchte vermieden wird.

Durch die beschriebenen Schutzeinrichtungen wird die direkte Übertragung von Keimen oder anderweitigen Verunreinigungen von der Hand des behandelnden Arztes auf das Bestrahlungsgerät verhindert, es wird jedoch nicht verhindert, daß andere Teile, beispielsweise die Lichtaustrittsfläche, sei es durch zufällige Berührung oder auch anderweitig kontaminiert werden. Eine derartige, nicht durch die Hand des behandelnden Arztes bedingte Kontaminierung wird jedoch bei solchen Bestrahlungsgeräten bedeutsam, die in unmittelbarer Nähe des Behandlungsortes, beispielsweise zur Ausleuchtung oder Bestrahlung innerhalb der Mundhöhle oder des Rachenraumes eingesetzt werden. Bei solchen Geräten erwächst insbesondere die Gefahr der Kontaminierung durch direkte Berührung mit dem Behandlungsort und durch Keimübertragung durch Blut oder andere Körperflüssigkeit.

Ausgehend von dem vorstehend beschriebenen Stand der Technik liegt der Erfindung die Aufgabe zugrunde, eine Schutzvorrichtung für ein medizinisches Bestrahlungsgerät bereitzustellen, die eine Übertragung von Keimen bzw. Verunreinigungen vom Patienten auf das Gerät sowie umgekehrt von dem Gerät auf den Patienten zuverlässig verhindert, ohne die Funktionsweise des Bestrahlungsgerätes wesentlich zu beeinträchtigen.

Die Erfindung wird für eine Schutzvorrichtung gemäß dem Oberbegriff des Anspruchs 1 dadurch gelöst, daß wenigstens an dem geschlossenen Ende des Hüllteils ein Fenster vorgesehen ist, das für Strahlung im Wellenlängenbereich von 370-520 nm wenigstens teilweise durchlässig ist. Eine solche Schutzvorrichtung kann mit dem Hüllteil prinzipiell über das gesamte Bestrahlungsgerät gezogen bzw. auf dieses aufgesteckt werden. Dadurch kann der eventuell mit dem Behandlungsort oder mit Körperflüssigkeit in Berührung kommende Bereich des Bestrahlungsgerätes optimal vor Kontamination geschützt werden und es wird gleichzeitig gewährleistet, daß das Bestrahlungsgerät nahe genug an den Behandlungsort herangeführt werden kann, damit die dem Bestrahlungsgerät entstammende Strahlung optimal auf den Behandlungsort eingerichtet werden kann. Durch die Durchlässigkeit für Strahlung im Wellenlängenbereich von 370-520 nm wird dem Behandlungsort sowohl Lichtstrahlung zur Beleuchtung des Behandlungsortes als auch solche Strahlung, die beispielsweise der Lichtpolymerisation von Kunststoffen für dentale Anwendungen dient, zugeführt. Die im Wellenlängenbereich zwischen 370 und 520 nm liegende Strahlung verhindert darüberhinaus sowohl eine Blendung des Patienten oder des Arztes (blaues Licht) als auch eine Aufheizung des Behandlungsortes durch Wärmestrahlung, in den Fällen, in denen diese nicht erwünscht ist.

Um eine Bestrahlung des Behandlungsortes mit kurzwelliger UV-Strahlung, insbesondere mit UVB-Strahlung zu vermeiden, ist es zweckmäßig, daß das Fenster ein optisches Filter bildet, das für Strahlung im Wellenlängenbereich unterhalb 370 nm undurchlässig ist. Dadurch wird insbesondere eine zu starke Belastung des Behandlungsortes durch die Strahlung vermieden.

Vorteilhafterweise ist das Fenster aus einem Kunststoff gebildet. Zweckmäßig ist es dabei, daß mindestens das Fenster aus einem Polyethylen oder Polypropylen, einem Polyterephtalat, einem Zelluloseproprionat, einem Polyester- und/oder Copolyester besteht. Zelluloseproprionat, Polyester bzw. Copolyester sind vorteilhaft einsetzbar, da diese Materialien sich in beträchtlichen Schichtdicken herstellen und verwenden lassen. Diese Materialien sind tiefziehbar, so daß formbeständige Fenster oder komplette Hüllteile herstellbar sind. Auch Polyethylen und Polypropylen lassen sich einsetzen, da diese eine hohe Dehn-und Reißfestigkeit aufweisen. Sie lassen sich daher als sehr dünne Folien herstellen, wobei diese Folien auch sehr gut geeignet sind, einfach in Form eines Folienbeutels über das Bestrahlungsgerät bzw. über die relevanten Teile des Bestrahlungsgerätes gezogen zu werden, wobei lediglich darauf geachtet werden muß, daß eine ggf. erforderliche Kühlung des Bestrahlungsgerätes nicht beeinträchtigt wird. Ein solcher Folienbeutel ist vorteilhaft, da sich die Folie im Bereich der Lichtaustrittsfläche des Bestrahlungsgerätes sehr gut an dessen Oberfläche anlegt.

Um eine einfache Herstellung der Schutzvorrichtung bzw. auch eine einfache Handhabung zu ermöglichen, ist es zweckmäßig, daß das gesamte Hüllteil aus einem für Strahlung im Wellenlängenbereich von 370-520 nm durchlässigen Material gebildet ist.

Um ein einwandfreies Anliegen des Fensters an der Lichtaustrittsfläche des Bestrahlungsgerätes zu gewährleisten, kann es zweckmäßig sein, daß das Fenster aus nichtdeformierbarem Material gebildet ist. In diesem Fall ist es vorteilhaft, daß das Fenster aus Borosilikatglas und/oder Quarzglas gebildet ist.

Um eine sichere Befestigung der Schutzvorrichtung an dem Bestrahlungsgerät zu gewährleisten, ist es zweckmäßig, daß die Mittel zur Halterung des Hüllteils über die Länge des Hüllteiles verteilt sind. Dadurch wird vermieden, daß sich das Hüllteil während der Handhabung des Bestrahlungsgerätes verschiebt und dadurch der Lichtdurchtritt durch das Fenster vermindert wird.

Besonders zweckmäßig ist es, daß die Mittel zur Halterung durch ein oder mehrere elastisch verformbare(s) ringförmigen(s) Spannelement(e) gebildet sind (ist). Durch elastisch verformbare Spannelemente kann die Schutzvorrichtung variabler auch bei unterschiedlichen Bestrahlungsgeräten eingesetzt werden, ohne daß die Befestigung des Hüllteils an dem Bestrahlungsgerät nachteilig beeinflußt wird. Der Einfachheit halber sind diese Spannelemente als O-Ringe ausgebildet.

Nachstehend wird die Erfindung an einem Ausführungsbeispiel anhand einer Zeichnung näher erläutert. In der Zeichnung zeigt
Figur 1 eine Schutzvorrichtung für das Lichtaustrittsteil eines medizinischen Bestrahlungsgerätes,
Figur 2 eine Schutzvorrichtung, die das Lichtaustrittsteil und die Bedienelemente eines medizinischen Bestrahlungsgerätes umfaßt,
Figur 3 eine Schutzvorrichtung für das Griffstück einschließlich des Lichtaustrittsteils eines Bestrahlungsgerätes und
Figur 4 die Anordnung eines Fensters an der Lichtaustrittsfläche eines Bestrahlungsgerätes.

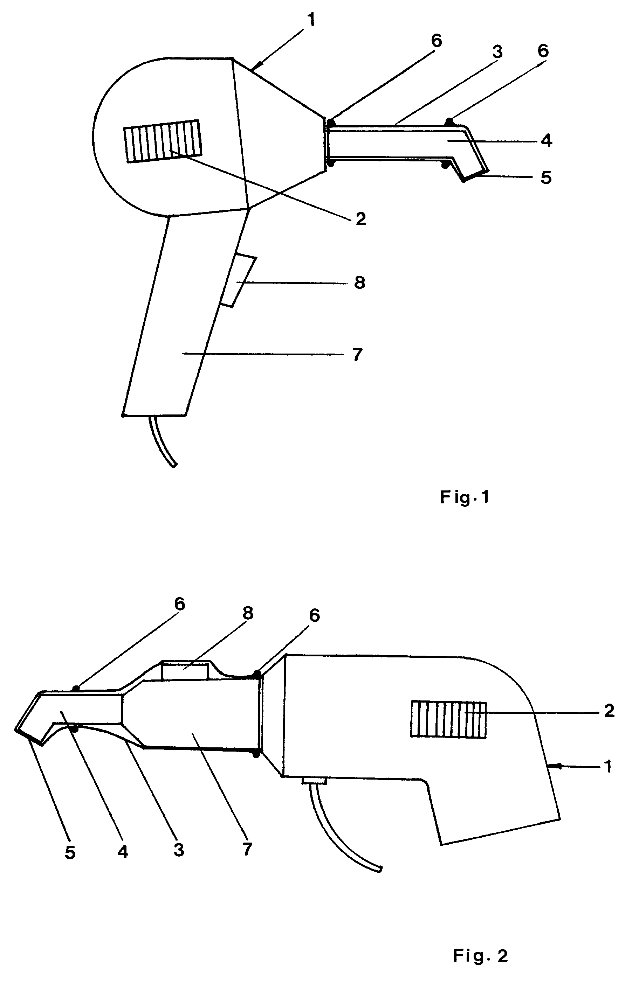

Bei Bestrahlungsgeräten, die in unmittelbarer Nähe des Behandlungsortes verwendet werden, wie beispielsweise Bestrahlungsgeräte für dentale Anwendungen oder auch Wundhaken, und die gleichzeitig die Strahlenquelle enthalten, ist es problematisch, die gesamte Geräteoberfläche mit einer Schutzvorrichtung zu bedecken, da dabei auch beispielsweise die für die Kühlung der Strahlenquelle notwendigen Lüftungsschlitze 2 abgedeckt würden. Beim einem solchen Bestrahlungsgerät 1 ist das Hüllteil 3 aus einer Polyethylenfolie über den als Lichtaustrittsteil dienenden Lichtleitstab 4 gezogen. Dabei liegt ein Teil der Polyethylenfolie direkt an der Lichtaustrittsfläche 5 des Lichtleitstabes 4 an. Ein Abgleiten des Hüllteiles 3 von dem Lichtleitstab 4 wird durch O-Ringe 6 verhindert. Ähnlich wie diese in Figur 1 gezeigte Schutzvorrichtung ist auch die in Figur 2 dargestellte Schutzvorrichtung gestaltet. Aufgrund der anders gearteten Form des Bestrahlungsgerätes 1 kann das Hüllteil 3 jedoch auch das Griffstück 7 mit dem Schalter 8 umfassen. Auch hier liegt die als Hüllteil verwendete Polyethylenfolie flächig an der Lichtaustrittsfläche 5 des Lichtleiter 4 an, so daß die durch das Fenster des Hüllteils 3 dringende Strahlung nur unwesentlich an Intensität verliert.

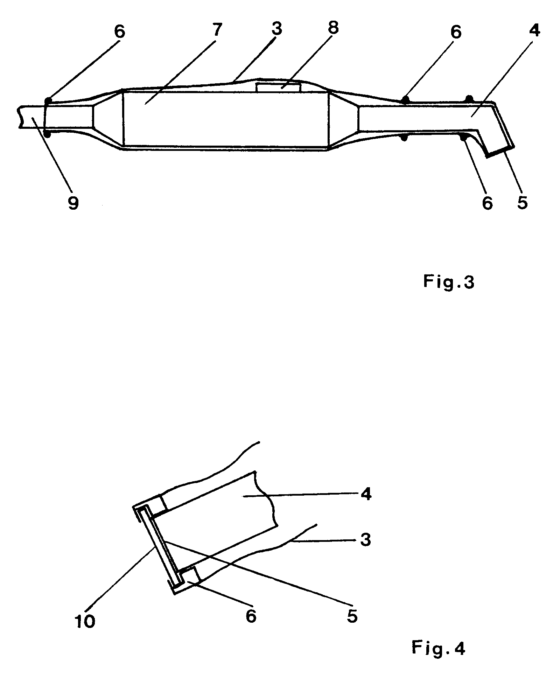

In Figur 3 ist eine Schutzvorrichtung für das Griffstück 7 eines Bestrahlungsgerätes 1 dargestellt. Bei diesem Bestrahlungsgerät 1 ist die Strahlenquelle durch einen Lichtleiter 9 mit dem Griffstück 7 verbunden. Hier ist das gesamte Griffstück 7 mit dem Lichtleitstab 4 von dem Hüllteil 3, das ebenfalls aus einer Polyethylenfolie gebildet ist, überzogen, wobei das Hüllteil 3 wiederum flächig an der Lichtaustrittsfläche 5 anliegt. Bei einer solchen Anordnung kann das Hüllteil 3 nahezu beliebig weit über das Griffstück 7 mit dem anschließenden Lichtleiter 9 gezogen werden, so daß die nicht durch das Hüllteil 3 geschützte Oberfläche des Bestrahlungsgerätes 1 weit genug vom Behandlungsort entfernt ist, um mit diesem nicht in Berührung zu kommen.

Figur 4 zeigt einen Teil einer Schutzvorrichtung, bei der an dem geschlossenen Ende des Hüllteils 3 ein nichtdeformierbares Fenster 10 aus Borosilikatglas angeordnet ist. Dieses Fenster 10 ist in einem Spannelement 6 gehaltert, welches gleichzeitig der Fixierung des Hüllteils 3 an dem Lichtleitstab 4 dient.

## Patentansprüche

1. Schutzvorrichtung für ein medizinisches Bestrahlungsgerät, die ein Hüllteil mit einer Aufnahmeöffnung und mit Mitteln zur Halterung des Hüllteils an einem Bestrahlungsgerät aufweist, dadurch gekennzeichnet, daß wenigstens an dem geschlossenen Ende des Hüllteils (3) ein Fenster vorgesehen ist, das für Strahlung im Wellenlängenbereich von 370-520 nm wenigstens teilweise durchlässig ist.

2. Schutzvorrichtung nach Anspruch 1 oder 2, dadurch gekennzeichnet, daß das Fenster ein optisches Filter bildet, das für Strahlung im Wellenlängenbereich unterhalb 370 nm undurchlässig ist.

3. Schutzvorrichtung nach Anspruch 1 oder 2, dadurch gekennzeichnet, daß das Fenster aus einem Kunststoff gebildet ist.

4. Schutzvorrichtung nach Anspruch 3, dadurch gekennzeichnet, daß mindestens das Fenster des Hüllteils (3) aus einem Polyethylen oder Polypropylen, einem Polyterephtalat, einem Cellulosepropionat, einem Polyester und/oder einem Copolyester besteht.

5. Schutzvorrichtung nach Anspruch 4, dadurch gekennzeichnet, daß mindestens das Fenster des Hüllteils (3) aus Cellulosepropionat oder einem Copolyester besteht.

6. Schutzvorrichtung nach Anspruch 4, dadurch gekennzeichnet, daß mindestens das Fenster des Hüllteils (3) aus Polyethylen bzw. Polypropylen besteht.

7. Schutzvorrichtung nach Anspruch 6, dadurch gekennzeichnet, daß das Fenster des Hüllteils (3) aus einer dünnen Folie gebildet ist.

8. Schutzvorrichtung nach Anspruch 3, dadurch gekennzeichnet, daß das gesamte Hüllteil (3) aus einem für Strahlung im Wellenlängenbereich von 370-520 nm durchlässigen Material gebildet ist.

9. Schutzvorrichtung nach Anspruch 1, dadurch gekennzeichnet, daß das Fenster aus nichtdeformierbarem Material (10) gebildet ist.

10. Schutzvorrichtung nach Anspruch 9, dadurch gekennzeichnet, daß das Fenster aus Borosilikatglas und/oder Quarzglas besteht.

11. Schutzvorrichtung nach einem der Ansprüche 1 bis 10, dadurch gekennzeichnet, daß die Mittel zur Halterung (6) des Hüllteils (3) an dem Bestrahlungsgerät (1) über die Länge des Hüllteiles (3) verteilt sind.

12. Schutzvorrichtung nach Anspruch 1 oder 11 , dadurch gekennzeichnet, daß die Mittel zur Halterung (6) durch ein oder mehrere elastisch verformbare(s) ringförmige(s) Spannelement(e) gebildet sind (ist).

13. Schutzvorrichtung nach Anspruch 12, dadurch gekennzeichnet, daß die Spannelemente (6) O-Ringe sind.
